# EUROPEAN PATENT APPLICATION

(11) **EP 4 659 722 A1**
(43) Date of publication of application: **10.12.2025**
(21) Application number: 23927550.6
(22) Date of filing: 13.04.2023
(51) Int. Cl.: A61F 13/15, A61F 13/514, A61F 13/551, B65D 85/07, B65D 85/18

(54) **PACKAGE, CONTAINER, ABSORBENT ARTICLE, AND METHOD FOR PRODUCING PACKAGE**

(30) Priority: 15.03.2023 JP 2023040748
(71) Applicant: Unicharm Corporation, Shikokuchuo-shi, Ehime 799-0111 (JP)
(72) Inventor: HASHINO, Akira, Kanonji-shi, Kagawa 769-1602 (JP); NISHIMURA, Kiyoko, Kanonji-shi, Kagawa 769-1602 (JP)
(74) Representative: Dolleymores
(86) International application number: PCT/JP2023/015050
(87) International publication number: WO 2024/189931

(57) **Abstract**

To provide an absorbent article and the like capable of exerting an adsorbing capability after the absorbent article is used. A package (1) includes an absorbent article (10) having a liquid-permeable topsheet, a liquid-impermeable backsheet, and an absorbent core disposed between the topsheet and the backsheet; and a packaging sheet (20) for individually packaging the absorbent article. The package includes an absorbent (50) that adsorbs at least one of an inorganic substance and an organic substance. The absorbent is separate from the absorbent article and is provided within a packaging space (WS) that contains the absorbent article in the package.

## Description

### [TECHNICAL FIELD]

The present invention is related to an absorbent article having an absorbent, a package in which an absorbent article is packaged, and a container containing therein a plurality of absorbent articles.

### [BACKGROUND ART]

Patent Literature 1 discloses an absorbent article having a moisture absorbent serving as an absorbent. The moisture absorbent in Patent Literature 1 is positioned between a topsheet and an absorbent core. The absorbent article is configured to exert a moisture absorbing capability on excreted fluid and to inhibit occurrence of discomfort such as steaminess, stickiness, or the like while being attached.

### [CITATION LIST]

### [PATENT LITERATURE]

[PATENT LITERATURE 1] Japanese Patent Laid-Open No. 2008-132280

### [SUMMARY OF INVENTION]

However, the aforementioned absorbent article has the following disadvantages.

The absorbent in Patent Literature 1 is positioned between the topsheet and the absorbent core and exerts an adsorbing capability in the process in which the excreted fluid is guided to the absorbent core. Generally speaking, absorbents such as moisture absorbents are configured to adsorb an inorganic substance or an organic substance. Once such a substance has been adsorbed, repeated adsorption becomes difficult. In other words, the adsorbing capability is gradually degraded by the use. In a mode in which the moisture absorbing capability is exerted in the process of absorbing body fluid, although the exertion of the adsorbing capability is possible during the use of the absorbent article, it is often the case that the adsorbing capability is degraded after the use of the absorbent article. Further, in a certain mode of use, an absorbent article after use may not be disposed of immediately, but may be disposed of after being kept for a while. In such a mode in which the absorbent article is kept after use, an adverse impact such as odor easily occurs during the keeping process.

For this reason, there is a demand for exertion of an adsorbing capability such as a deodorizing capability on an absorbent article after use.

The present invention is made in view of the disadvantages described above and provides an absorbent article and the like capable of exerting an adsorbing capability after the absorbent article is used.

A package according to one aspect includes: an absorbent article having a liquid-permeable topsheet, a liquid-impermeable backsheet, and an absorbent core disposed between the topsheet and the backsheet; and a packaging sheet for individually packaging the absorbent article. The package includes an absorbent that adsorbs at least one of an inorganic substance and an organic substance. The absorbent is separate from the absorbent article and is provided within a packaging space that contains the absorbent article in the package.

A container according to one aspect includes: an absorbent article having a liquid-permeable topsheet, a liquid-impermeable backsheet, and an absorbent core disposed between the topsheet and the backsheet; and a container sheet containing a plurality of the absorbent articles. The container includes an absorbent that adsorbs at least one of an inorganic substance and an organic substance. The absorbent is separate from the absorbent article and is provided within a containing space that contains the absorbent articles in the container.

An absorbent article according to one aspect includes a liquid-permeable topsheet, a liquid-impermeable backsheet, and an absorbent core disposed between the topsheet and the backsheet. The absorbent article includes an absorbent that adsorbs at least one of an inorganic substance and an organic substance. The absorbent is provided on a non-skin side relative to the backsheet.

An absorbent article according to another aspect includes a liquid-permeable topsheet, a liquid-impermeable backsheet, and an absorbent core disposed between the topsheet and the backsheet. The absorbent article includes an absorbent that adsorbs at least one of an inorganic substance and an organic substance. The absorbent article has a flap positioned on a non-skin side relative to the backsheet when being worn. The absorbent is provided on the flap.

A method for manufacturing a package according to one aspect is a method for manufacturing a package having: an absorbent article having a fastening portion to be fastened to a wearable article when being worn; a stripping sheet that covers the fastening portion; and a packaging sheet joined to a surface of the stripping sheet on a side opposite to the absorbent article, for individually packaging the absorbent article. The method for manufacturing the package includes: an adhesion application step of providing a plurality of joining regions to which an adhesive is applied on one of the stripping sheet and the packaging sheet; an absorbent joining step of joining an absorbent to a part of the plurality of joining regions; and a sheet joining step of joining the stripping sheet and the packaging sheet via other joining regions among the plurality of joining regions.

### [BRIEF DESCRIPTION OF DRAWINGS]

[FIG. 1] FIG. 1 is a diagram showing a package in a packaged state according to a first embodiment. FIG. 1(a) is a plan view, and FIG. 1(b) is a schematic cross-sectional view taken along line A-A shown in FIG. 1(a).
[FIG. 2] FIG. 2 is a plan view of the package in an expanded state as seen from the skin side.
[FIG. 3] FIG. 3 is a schematic cross-sectional view taken along line B-B shown in FIG. 2.
[FIG. 4] FIG. 4 is a diagram for explaining a manner of taking out an absorbent article from the package according to the embodiment.
[FIG. 5] FIG. 5 is a perspective view of a container according to a second embodiment.
[FIG. 6] FIG. 6 is a cross-sectional view of an absorbent article according to a third embodiment.
[FIG. 7] FIG. 7 is a plan view of an absorbent article according to a fourth embodiment as seen from the skin side.

### [DESCRIPTION OF EMBODIMENTS]

### (1) Summary of Embodiments

At least the following matters will become apparent from the descriptions in this specification and the accompanying drawings.

A package is the invention according to Aspect 1. The package includes: an absorbent article having a liquid-permeable topsheet, a liquid-impermeable backsheet, and an absorbent core disposed between the topsheet and the backsheet; and a packaging sheet for individually packaging the absorbent article. The package includes an absorbent that adsorbs at least one of an inorganic substance and an organic substance. The absorbent is separate from the absorbent article and is provided within a packaging space that contains the absorbent article in the package. According to this aspect, by re-containing the used absorbent article and the absorbent in the packaging space for disposal, the absorbent can exert its adsorbing capability on the absorbent article. Furthermore, because the absorbent is separate from the absorbent article, it is unlikely to exert its adsorbing capability on the absorbent article during use. Therefore, the adsorbing capability of the absorbent is maintained, and it can exert its adsorbing capability on the used absorbent article.

According to a preferred aspect, the invention according to Aspect 2 may have the following features in the invention according to Aspect 1. The absorbent article has a fastening portion to be fastened to a wearable article when being worn. The package includes a stripping sheet disposed between the absorbent article and the packaging sheet, which covers the fastening portion before the absorbent article is used. The absorbent is disposed between the stripping sheet and the packaging sheet. According to this aspect, before the absorbent article is used, the stripping sheet is disposed between the absorbent and the absorbent article. Therefore, the absorbent is unlikely to exert its adsorbing capability on the absorbent article before use, and a decrease in the adsorbing capability can be suppressed.

According to a preferred aspect, the invention according to Aspect 3 may have the following features in the invention according to Aspect 1 or Aspect 2. The absorbent is not joined to the stripping sheet but is joined to the packaging sheet. According to this aspect, the absorbent remains within the packaging space, which can suppress unintentional dislodging of the absorbent, and by containing the used absorbent article in the packaging space, both the absorbent and the absorbent article are placed within the packaging space, allowing the absorbent to exert its adsorbing capability on the used absorbent article.

According to a preferred aspect, the invention according to Aspect 4 may have the following features in the invention according to Aspect 1 or Aspect 2. The absorbent is not joined to the packaging sheet but is joined to the stripping sheet. According to this aspect, the absorbent remains within the packaging space, which can suppress unintentional dislodging of the absorbent. In addition, by containing the used absorbent article in the packaging space, both the absorbent and the absorbent article are placed within the packaging space, allowing the absorbent to exert its adsorbing capability on the used absorbent article.

According to a preferred aspect, the invention according to Aspect 5 may have the following features in the invention according to any one of Aspects 2 to 4. In a region where the packaging sheet and the stripping sheet overlap, a joining region where the packaging sheet and the stripping sheet are joined, and a non-joining region where the packaging sheet and the stripping sheet are not joined, are provided. The absorbent is disposed so as to overlap with the non-joining region. When taking out the absorbent article from the packaging space, a user generally grips a part of the absorbent article and pulls it out of the packaging space. At this time, the stripping sheet is pulled together with the absorbent article via the fastening portion. The stripping sheet is joined to the packaging sheet in the joining region and cannot be separated from the packaging sheet, whereas in the non-joining region, it can be separated from the packaging sheet. In the non-joining region, the stripping sheet is likely to lift, and the absorbent overlaps with this non-joining region. Therefore, in the state after the absorbent article has been taken out, a space is created between the stripping sheet and the packaging sheet, making it easier for the absorbent to exert its adsorbing capability, and thus allowing it to exert its adsorbing capability more effectively on the used absorbent article.

According to a preferred aspect, the invention according to Aspect 6 may have the following features in the invention according to Aspect 5. The absorbent article is folded in half with a fold line extending in the width direction as a reference point. An outlet for taking out the absorbent article in the package is configured to be provided at an end of the package in the front-rear direction, which is opposite to the fold line. The joining region includes a first joining region and a second joining region located on both sides in the front-rear direction across the fold line. The non-joining region is continuously provided between the first joining region and the second joining region. The fastening portion and the absorbent are provided so as to overlap with the non-joining region. According to this aspect, the stripping sheet can be pulled out via the fastening portion in the continuous non-joining region between the first and second joining regions. The non-joining region overlaps with the fold line, allowing the fold line portion of the stripping sheet to be separated from the packaging sheet. This creates a wider space around the absorbent when the absorbent article is taken out, allowing the absorbent to exert its adsorbing capability more effectively on the used absorbent article.

According to a preferred aspect, the invention according to Aspect 7 may have the following features in the invention according to any one of Aspects 1 to 6. The packaging sheet is a sheet member with an oxygen transmission rate in the thickness direction of 200 cc/m²/day/atm or less. According to this aspect, since the packaging sheet has a low oxygen transmission rate, the sealing performance of the packaging space in the packaged state is maintained, the degradation of the absorbent's adsorbing capability is suppressed, and the adsorbing capability can be more effectively exerted on the used absorbent article.

According to a preferred aspect, the invention according to Aspect 8 may have the following features in the invention according to any one of Aspects 1 to 7 and Aspects 9 to 18. The absorbent article is folded with a fold line as a reference point before use. The absorbent is disposed in a region that does not overlap with the fold line. According to this aspect, damage to the absorbent caused by being folded along the fold line is suppressed, and the adsorbing capability of the absorbent can be exerted.

According to a preferred aspect, the invention according to Aspect 9 may have the following features in the invention according to any one of Aspects 1 to 8 and Aspects 10 to 18. The absorbent article comprises a functional agent including at least one of a testing member for examining a user's health condition, an antibacterial agent, and an antiviral agent. According to this aspect, before the use of the absorbent article, the adsorbing capability of the absorbent can suppress the degradation of the functional agent's performance.

According to a preferred aspect, the invention according to Aspect 10 may have the following features in the invention according to any one of Aspects 1 to 9 and Aspects 11 to 18. The absorbent core is disposed between the functional agent and the absorbent. The absorbent core comprises an absorbent material such as pulp and is likely to function as a cushioning material. The absorbent core disposed between the functional agent and the absorbent functions as a cushioning material and can suppress unintentional damage to the functional agent and the absorbent.

The invention according to Aspect 11 is a container. The container includes: an absorbent article having a liquid-permeable topsheet, a liquid-impermeable backsheet, and an absorbent core disposed between the topsheet and the backsheet; and a container sheet containing a plurality of the absorbent articles. The container includes an absorbent that adsorbs at least one of an inorganic substance and an organic substance. The absorbent is separate from the absorbent article and is provided within a containing space that contains the absorbent articles in the container. According to this aspect, since the absorbent is provided within the containing space, it can exert its adsorbing capability on the used absorbent article. Furthermore, the absorbent is separate from the absorbent article and does not exert its adsorbing capability on the body fluid absorbed by the absorbent article during use. Therefore, it can exert its adsorbing capability on the used absorbent article placed in the containing space.

According to a preferred aspect, the invention according to Aspect 12 may have the following features in the invention according to Aspect 11. The container sheet is a sheet member with an oxygen transmission rate in the thickness direction of 200 cc/m²/day/atm or less. Since the container sheet has a low oxygen transmission rate, the sealing performance of the containing space is maintained, the degradation of the absorbent's adsorbing capability is suppressed, and the adsorbing capability can be exerted on the used absorbent article.

The invention according to Aspect 13 is an absorbent article according to one aspect. An absorbent article according to one aspect includes a liquid-permeable topsheet, a liquid-impermeable backsheet, and an absorbent core disposed between the topsheet and the backsheet. The absorbent article includes an absorbent that adsorbs at least one of an inorganic substance and an organic substance. The absorbent is provided on a non-skin side relative to the backsheet. Excreted body fluid is guided from the topsheet to the absorbent core and absorbed by the absorbent core, and is unlikely to be guided to the non-skin side of the backsheet during the use of the absorbent article. The absorbent is disposed with the absorbent core and the backsheet interposed therebetween, is unlikely to come into contact with body fluid during use, and is unlikely to exert its adsorbing capability. Therefore, even in the used absorbent article, the degradation of the absorbent's adsorbing capability is suppressed, and the adsorbing capability can be exerted.

According to a preferred aspect, the invention according to Aspect 14 may have the following features in the invention according to Aspect 13. The absorbent article includes a functional agent including at least one of a testing member for examining a user's health condition, an antibacterial agent, and an antiviral agent. The functional agent is disposed on a skin side relative to the backsheet. According to this aspect, the functional agent and the absorbent are disposed with the backsheet interposed therebetween. The backsheet can prevent contact between the functional agent and the absorbent, making it easier to maintain the performance of both.

According to a preferred aspect, the invention according to Aspect 15 may have the following features in the invention according to Aspect 13 or Aspect 14. The absorbent is a member constituting a non-skin surface of the absorbent article. According to this aspect, the adsorbing capability of the absorbent is easily exerted with the non-skin surface of the absorbent article facing outward. For example, it is easy to exert the adsorbing capability when the absorbent article is rolled up with its skin side inward for disposal.

The invention according to Aspect 16 is an absorbent article according to another aspect. An absorbent article according to another aspect includes a liquid-permeable topsheet, a liquid-impermeable backsheet, and an absorbent core disposed between the topsheet and the backsheet. The absorbent article includes an absorbent that adsorbs at least one of an inorganic substance and an organic substance. The absorbent article has a flap positioned on a non-skin side relative to the backsheet when being worn. The absorbent is provided on the flap. The flap is positioned on the non-skin side relative to the backsheet during use. The absorbent is unlikely to come into contact with body fluid during use and is unlikely to exert its adsorbing capability. Therefore, even in the used absorbent article, the degradation of the absorbent's adsorbing capability is suppressed, and it can exert its adsorbing capability on the used absorbent article.

According to a preferred aspect, the invention according to Aspect 17 may have the following features in the invention according to Aspect 16. The absorbent is provided only on the flap. The absorbent is provided only on the flap, which is unlikely to come into contact with body fluid during use, thereby suppressing the exertion of its performance during the use of the absorbent article. Therefore, the adsorbing capability of the absorbent can be exerted after use.

According to a preferred aspect, the invention according to Aspect 18 may have the following features in the invention according to Aspect 16 or Aspect 17. The flap is folded to cover a skin-side surface of the absorbent article before use. At least a part of the absorbent is disposed on a skin surface of the flap in a folded region of the flap. Before the use of the absorbent article, the absorbent is sandwiched between the folded region of the flap and an unfolded region (a region other than the flap) of the absorbent article. Therefore, deterioration and dislodging of the absorbent before use can be suppressed.

The invention according to Aspect 19 is a method for manufacturing a package according to one aspect. The method for manufacturing a package is a method for manufacturing a package including: an absorbent article having a fastening portion to be fastened to a wearable article when being worn; a stripping sheet that covers the fastening portion; and a packaging sheet joined to a surface of the stripping sheet on a side opposite to the absorbent article, for individually packaging the absorbent article. The method for manufacturing the package comprises: an adhesion application step of providing a plurality of joining regions to which an adhesive is applied on one of the stripping sheet and the packaging sheet; an absorbent joining step of joining an absorbent to a part of the plurality of joining regions; and a sheet joining step of joining the stripping sheet and the packaging sheet via other joining regions among the plurality of joining regions. According to this aspect, in the adhesion application step, it is possible to provide both the joining region for joining the absorbent and the joining region for joining the stripping sheet and the packaging sheet. This can simplify the manufacturing process compared to a mode where each joining region is provided in a separate step.

### (2) First Embodiment (An embodiment of a package)

The following will describe a first embodiment of the present invention, with reference to the drawings. The first embodiment is related to a package 1. In the description of the drawings below, the same or similar parts are indicated with the same or similar reference numerals. It should be noted that the drawings are schematic and that proportions of the dimensions and the like may be different from those in reality. Thus, specific dimensions and the like should be determined by taking the following explanations into consideration. Further, there may be some parts where relationships and proportions of dimensions vary among the drawings. In the cross-sectional views, although the constituent elements are indicated apart from one another for the sake of convenience in the explanations, the constituent elements in actual products are in contact with one another.

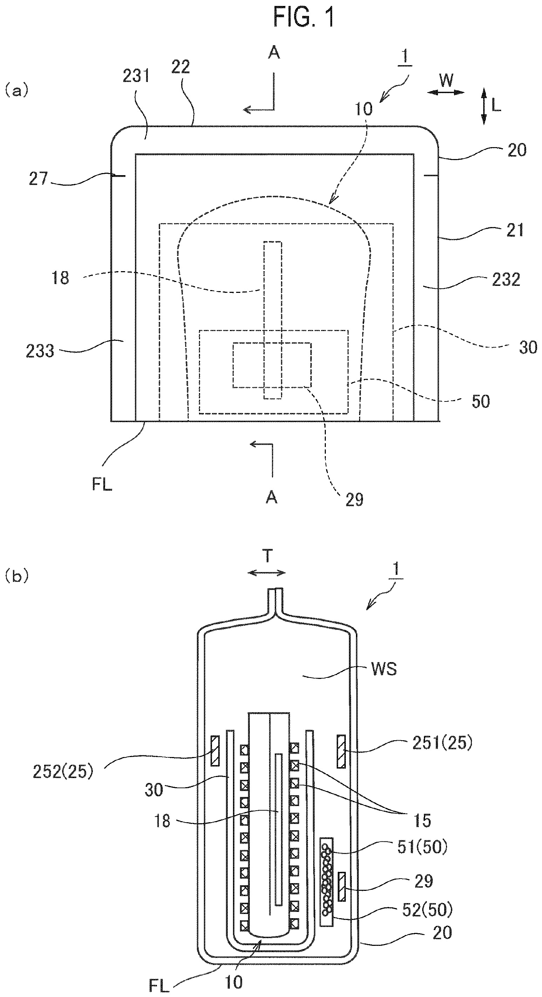

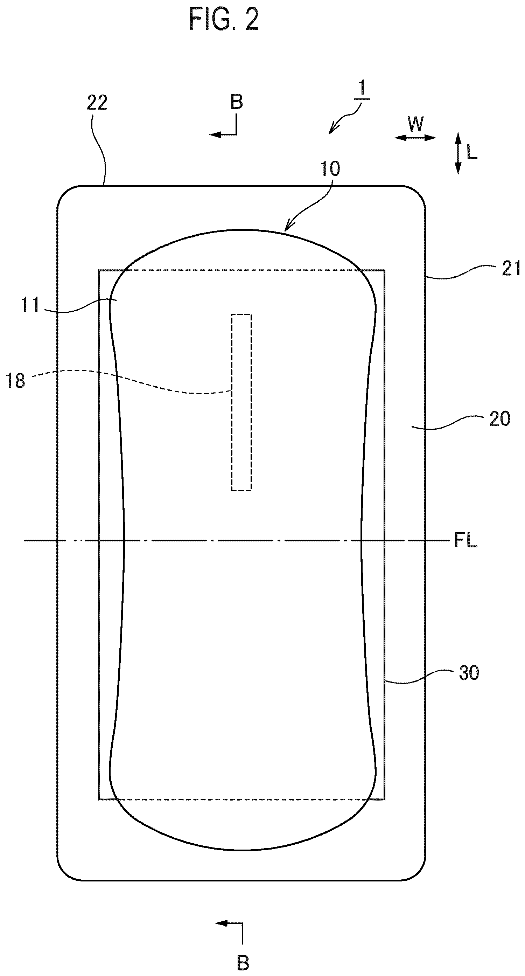

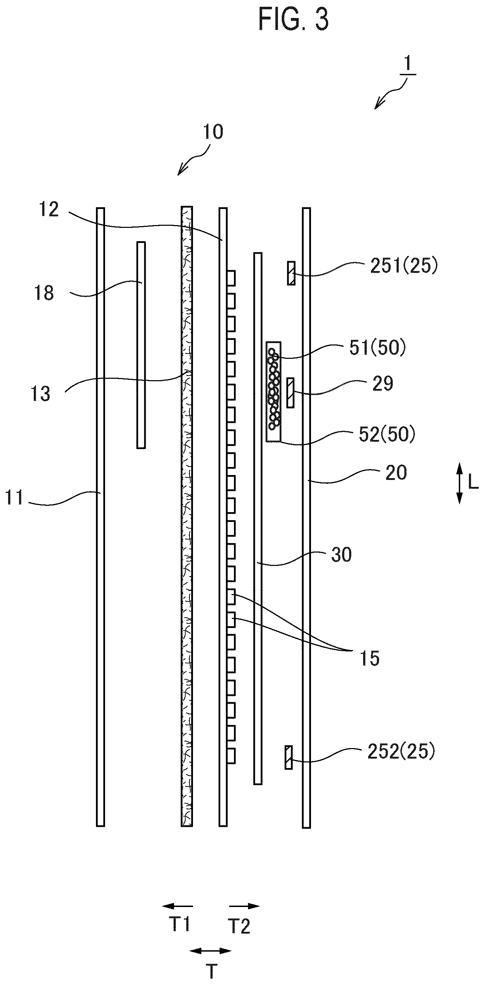

FIG. 1 is a drawing showing a packaged state of the package 1 according to the embodiment. FIG. 1(a) is a plan view. FIG. 1(b) is a schematic cross-sectional view taken along line A-A indicated in FIG. 1. FIG. 2 is a plan view of the package 1 in an expanded state, as seen from a skin side T1. FIG. 3 is a cross-sectional view taken along line B-B indicated in FIG. 2. The package 1 has a front-rear direction L, a width direction W, and a thickness direction T that are orthogonal to one another and each extend along a plane direction. The front-rear direction L extends along a front-rear direction L of an absorbent article 10. The side that comes into contact with the lower abdomen of the user is referred to as a "front side" and the side that comes into contact with the buttocks of the user is referred to as a "rear side". The thickness direction T extends toward the skin side T1 and a non-skin side T2. The "skin side" represents the side that faces the wearer's skin during use. The "non-skin side" represents the side that faces away from the wearer's skin during use.

The package 1 includes at least the absorbent article 10, a packaging sheet 20 that packages the absorbent article 10, and an absorbent 50. The absorbent 50 is configured so that adsorption is realized by at least one of an inorganic substance and an organic substance and is configured to exert an adsorbing capability such as a deodorizing capability or a moisture absorbing capability. Examples thereof include desiccants and deodorants. It is possible to use a publicly known substance as the absorbent 50. The adsorption may be physical adsorption or chemical adsorption. For example, it is possible to use any of the following: silica gel (silicon dioxide), quicklime (calcium oxide), slaked lime, calcium chloride, activated clay, a silica-magnesia preparation, and silica. The package of the present embodiment is configured to exert an adsorbing capability on an absorbent article after use. In a preferable example, the absorbent may be solid. It is desirable that the absorbent is not a masking agent (an odor eliminator) using gas. The absorbent 50 may be in a granular form. The absorbent 50 in the present embodiment is represented by granular spherical bodies 51, a plurality of which are contained in an adsorption bag 52. When the absorbent 50 in the form of the spherical bodies 51 is used, because there are no sharp corners, it is possible to prevent the absorbent article 10, the packaging sheet, and the like from being damaged. Further, the absorbent 50 may be composed of only the spherical bodies 51 having an adsorbing capability. However, by having the spherical bodies 51 contained in the adsorption bag 52, it is possible to properly position the absorbent 50 at the time of manufacturing. Further, at the time of taking out the absorbent article 10 or the like, it is possible to inhibit inconveniences such as a part of the absorbent 50 falling out and to keep the absorbent 50 placed in an appropriate position. Also, the user is able to easily handle the absorbent 50. Alternatively, the absorbent may be in a liquid form that can be applied.

The absorbent article 10 may be an absorbent article such as a sanitary napkin, a panty liner, an incontinence pad, an excrement pad, a nursing breast pad, a disposable diaper, an underwear-type napkin, or the like. The absorbent article 10 may be an article used while being attached to a wearable article such as underwear or may be an absorbent article used without being attached to a wearable article. The absorbent article 10 in the embodiment is a panty liner used while being attached to a wearable article.

The absorbent article 10 may include a topsheet 11 that is liquid permeable, a backsheet 12 that is liquid impermeable, and an absorbent core 13. The topsheet 11 is a sheet that is positioned on the skin side T1 relative to the absorbent core 13 and that comes into contact with the wearer. The topsheet 11 may be made of an arbitrary sheet-like material having a structure that passes liquid, such as a non-woven fabric, a woven fabric, a porous plastic sheet, or a mesh sheet. The backsheet 12 is a sheet that is positioned on the non-skin side T2 relative to the absorbent core 13 and that comes into contact with a wearable article. As the backsheet 12, it is possible to use a polyethylene sheet, a laminated non-woven fabric mainly using polypropylene or the like, a breathable resin film, or a sheet obtained by joining a breathable resin film with a non-woven fabric such as a spunbond or a spunlace.

As shown in FIG. 3 etc., the absorbent article 10 has fastening portions 15 for fastening the absorbent article 10 to a wearable article such as underwear. The fastening portions 15 are regions positioned on the non-skin surface of the backsheet 12 and provided with fastening means (e.g., an adhesion agent) for fastening the absorbent article 10 onto the wearable article such as underwear. In the present embodiment, the plurality of fastening portions 15 are provided at intervals in the front-rear direction L, while each extending in the width direction W. Alternatively, in a modification example, the plurality of fastening portions 15 may be provided at intervals in the width direction W, while each extending in the front-rear direction L.

The absorbent core 13 is positioned between the topsheet 11 and the backsheet 12. The absorbent core 13 may be made of an absorptive material that absorbs liquid. Examples of the absorptive material for constituting the absorbent core 13 can include pulp and superabsorbent polymer (SAP), for example. The absorbent core 13 includes at least pulp. The absorbent article 10 may have a core wrap sheet (not shown) covering the absorbent core 13. The core wrap sheet can be made from non-woven fabric or tissue, for example.

The packaging sheet 20 is a sheet that individually packages the absorbent article and may be made of non-woven fabric or film, for example. To ensure sealability of the package 1, it is desirable that the packaging sheet 20 has low breathability and may be, in a preferable example, made of film. In an expanded state, the packaging sheet 20 is rectangle and has a pair of front-rear lateral sides 21 extending along the front-rear direction L and a pair of width lateral sides 22 extending along the width direction W. The front-rear lateral sides 21 structure an outer edge of the packaging sheet 20. The width lateral sides 22 structure an outer end edge of the packaging sheet 20. The package 1 of the present embodiment includes a stripping sheet 30 positioned between the absorbent article 10 and the packaging sheet 20. Prior to use of the absorbent article 10, the stripping sheet 30 covers the fastening portions 15. In a modification example, the package 1 does not necessarily need to include the stripping sheet 30. In a mode in which the stripping sheet 30 is not included, the packaging sheet 20 may cover the fastening portions 15 of the absorbent article 10. The stripping sheet 30 may include a paper base member and a releasing agent applied to the base member. The releasing agent may be a substance capable of lowering a friction coefficient of the sheet surface. Examples thereof include silicone-based resin, fluorine-based resin, and tetrafluoroethylene-based resin.

The packaging sheet 20 and the stripping sheet 30 may be joined together in joining regions 25. The plurality of joining regions 25 are provided at intervals in the front-rear direction L. The joining regions 25 of the present embodiment include a first joining region 251 overlapping with a front end of the stripping sheet 30 and a second joining region 252 overlapping with a rear end of the stripping sheet 30. The first joining region 251 and the second joining region 252 may be located on either side, in the front-rear direction L, of a fold line FL. Between the first joining region 251 and the second joining region 252, a non-joining region may be provided in an uninterrupted manner. The region other than the first joining region 251 and the second joining region 252 is the non-joining region where the packaging sheet 20 and the stripping sheet 30 are not joined together. The non-joining region includes not only the region between the first joining region 251 and the second joining region 252, but also a region on the front side of the first joining region 251, a region on the rear side of the second joining region 252, and regions on the outer sides, in the width direction W, of the joining regions 25.

In this situation, an outer portion in the present invention refers to a part that extends over a certain range in the width direction W, including the outer edge in the width direction W. The outer edge refers to an outer edge in the width direction W.

Further, the outer edge of any constituent element extending over a certain range in the front-rear direction refers to an edge obtained by connecting points positioned on outer sides, in the width direction, of the constituent element, throughout the entire constituent element. An inner portion in the present invention refers to a part that extends over a certain range in the width direction W, including an interior edge in the width direction W. The inner edge refers to an interior edge in the width direction W. Further, the inner edge of any constituent element extending over a certain range in the front-rear direction refers to an edge obtained by connecting points positioned on inner sides, in the width direction, of the constituent element throughout the entire constituent element. The front end and the rear end in the present invention each refer to a part that extends over a certain range in the front-rear direction L, including an edge in the front-rear direction L. A front end edge and a rear end edge each refer to an edge in the front-rear direction L. An outer end includes the front end and the rear end. The outer end edge includes a front end edge and a rear end edge. Further, an inner lateral side is a side that extends along the front-rear direction L, while including the inner edge. An outer lateral side is a side that extends along the front-rear direction L, while including the outer edge. In the present disclosure, the expression "along the front-rear direction L" denotes a direction oriented at an angle of 45° or smaller with respect to the front-rear direction L. The expression "along the width direction W" denotes a direction oriented at an angle of 45° or smaller with respect to the width direction W.

As a result of the packaging sheet 20 and the absorbent article 10 being folded together, the package 1 is brought into a packaged state in which the absorbent article 10 is individually packaged by the packaging sheet 20. Further, in a mode in which the stripping sheet 30 is provided, the stripping sheet 30 may also be folded together. The packaging sheet 20 and the absorbent article 10 may be folded into two, while using the fold line FL extending along the width direction W as a reference point. The fold line FL is a single line and may be located in the vicinity of the center, in the front-rear direction L, of the absorbent article 10. In the folded state using the fold line FL as the reference point, the packaging sheet 20 has a substantially rectangular shape defined by four sides. Among the four sides, the three sides other than the side formed by the fold line FL are provided with package joining portions in which mutually overlapping layers of the packaging sheet 20 are joined with each other. The package joining portions include: a first package joining portion 231 where the width lateral sides 22 are joined with each other; a second package joining portion 232 where the front-rear lateral sides 21 located on one side with respect to the center of the packaging sheet 20 in the width direction W are joined with each other; and a third package joining portion 233 where the front-rear lateral sides 21 located on the other side with respect to the center of the packaging sheet 20 in the width direction are joined with each other. The width of each of the package joining portions may be set as appropriate, but 5 mm or more is desirable to ensure sealability.

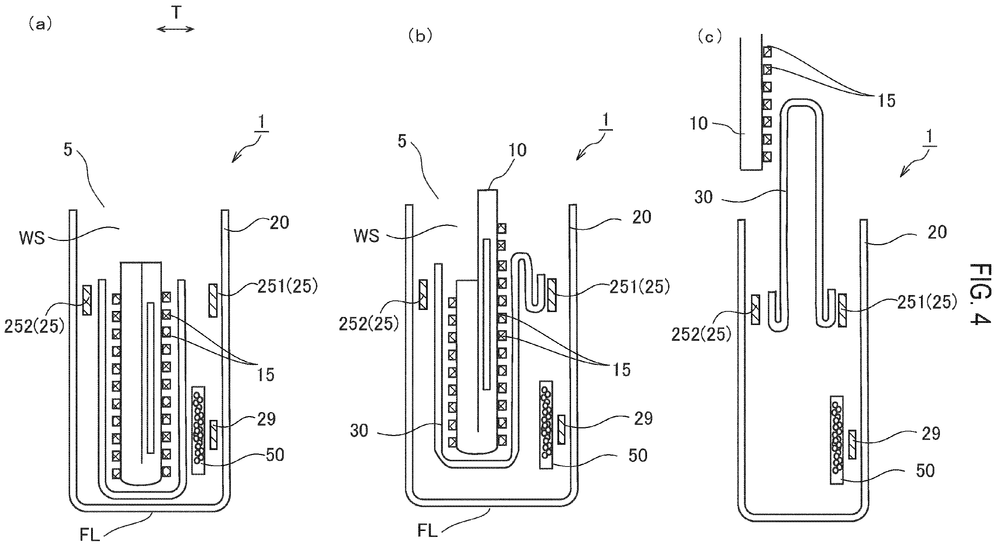

For the package joining portions, it is possible to use a well-known method such as fusion bonding, an adhesive agent, or the like. One end of the second package joining portion 232 may be contiguous with the first package joining portion 231. The other end of the second package joining portion 232 may be contiguous with the fold line FL. Further, one end of the third package joining portion 233 may be contiguous with the first package joining portion 231. The other end of the third package joining portion 233 may be contiguous with the fold line FL. As a result of the package joining portions being provided in the contiguous manner, the package 1 is brought into a sealed state. In the packaged state, the package 1 has formed therein a package space WS capable of containing the absorbent article 10 therein. The package space WS is a space interposed between inner surfaces of the packaging sheet 20 and, in a planar view, enclosed by the fold line FL, the first package joining portion 231, the second package joining portion 232, and the third package joining portion 233. In the packaged state, the packaging sheet 20 has formed therein incision lines 27 for forming an outlet through which the absorbent article 10 can be taken out. In the present embodiment, each of the incision lines 27 extends from a different one of the front-rear lateral sides 21 toward the inner side in the width direction W. The incision lines 27 are formed within the region where the first package joining portion 231 and the second package joining portion 232 are provided. As a result of the user cutting the packaging sheet 20 while using the incision lines 27 as a reference point, at least one of the first package joining portion 231 and the second package joining portion 232 is cut, so that the package space WS is opened up, and an outlet (see FIG. 4) 5 is formed. Of the ends, in the front-rear direction L, of the package 1 in the packaged state, the outlet 5 may be provided at the one end positioned on the opposite side from the fold line FL. With this configuration, at the time of taking out the absorbent article 10, the user is able to take out the absorbent article 10, by gripping either the front end or the rear end of the absorbent article 10, which is positioned on the opposite side from the fold line FL.

FIG. 4 is a schematic drawing of an example of a manner in which the absorbent article 10 is taken out of the package space WS of the package 1. When taking the absorbent article 10 out of the package 1, the user cuts a part of the packaging sheet 20 in the packaged state so as to form the outlet 5. FIG. 4(a) shows a state in which the outlet 5 has been formed. Inserting his/her fingers or the like through the outlet 5, the user grips and pulls the absorbent article 10 to the outside of the package space WS. FIG. 4(b) shows a state in which, a part (the front end) of the absorbent article has been pulled and moved toward the outside of the package space WS. When the user further pulls the absorbent article 10, the absorbent article 10 is taken out of the package space WS. FIG. 4(c) shows a state in which the absorbent article 10 has been pulled while both the front end and the rear end of the absorbent article are gripped, so that the absorbent article is taken out to the outside of the package space WS. In this situation, because the absorbent article 10 and the stripping sheet 30 are joined together via the fastening portions 15, it is feared that the stripping sheet 30 might be pulled to the outside of the package space WS together with the absorbent article 10. However, because the stripping sheet 30 and the packaging sheet 20 are joined together via the joining regions 25, the stripping sheet 30 does not get separated, together with the absorbent article 10, from the packaging sheet 20. Thus, the state in which the stripping sheet 30 and the packaging sheet 20 are joined together is maintained. Further, possible manners for taking out the absorbent article are not limited to the mode indicated in FIG. 4. For example, it is also acceptable to take out the absorbent article by gripping one of the front end and the rear end of the absorbent article and pulling the absorbent article. According to the above manner, the joining of the joining region located on the pulled side (for example, the first joining region 251 in the case where the front end is pulled) is cancelled, so that the stripping sheet 30 is separated from the packaging sheet 20, and one of the sides (e.g., the front end side) of the stripping sheet may be pulled to the outside of the package space, while the other side of the stripping sheet may remain in the package space.

The package 1 in the embodiment is configured to be able to exert the adsorbing capability on the absorbent article 10 after use. The absorbent 50 is a separate body from the absorbent article 10 and is provided in the package space WS that, in the package 1, contains the absorbent article 10 therein. Before the absorbent article 10 is used (before the package 1 is unsealed), the absorbent article 10 and the absorbent 50 are contained together in the package space WS. Accordingly, it is possible to have an adsorption effect exerted on the absorbent article 10 before use. In addition, at the time of replacing an absorbent article 10 and disposing of the absorbent article 10 having been used, it is general practice for some users to put the used absorbent article in the package space WS in which a new absorbent article 10 to be used was packaged. In that situation, because the absorbent 50 and the absorbent article 10 are contained together in the package space WS, it is possible to have the adsorbing capability exerted on the absorbent article 10 after use. Further, the absorbent 50 is a separate body from the absorbent article 10 and, during use, exerts no adsorbing capability on the body fluid absorbed by the absorbent article 10. Accordingly, it is possible to maintain the absorbing capability of the absorbent 50 and to have the adsorbing capability exerted on the absorbent article 10 after use. Consequently, it is possible to have the adsorbing capability effectively exerted on the absorbent article 10 after use that is positioned in the package space WS.

The absorbent 50 may be joined with the interior of the package space WS. It is sufficient as long as the absorbent 50 is joined so as to remain in the package space WS in a normal manner of use in which the absorbent article 10 is taken out of the package 1. A concept is that the absorbent 50 may be taken out of the package space WS if excessive force is applied. The absorbent 50 may be joined with the packaging sheet 20 or may be joined with the stripping sheet 30. As a result of the absorbent 50 being joined with the interior of the package space WS, it is possible to prevent the absorbent 50 from inadvertently coming out of the package space WS at the time of taking out the absorbent article 10 or the like. It is therefore possible to have the adsorption effect exerted on the absorbent article 10 after use that is contained in the package space WS.

It is sufficient as long as the absorbent 50 is a separate body from the absorbent article 10. The absorbent 50 may be joined with the absorbent article 10. Further, in the mode in which the absorbent 50 is joined with the absorbent article 10, the absorbent 50 may detachably be joined with the absorbent article 10 so that the absorbent 50 can be separated during the use of the absorbent article 10. It is desirable to position the absorbent 50 so as to be out of contact with the surface (the topsheet) of the absorbent article 10 that will touch the skin. For example, the absorbent 50 may temporarily be joined with the non-skin surface of the absorbent article 10 (the non-skin surface of the backsheet). By arranging the absorbent 50 in the position that is out of contact with the skin surface of the absorbent article 10, it is possible to prevent the surface of the absorbent article that will touch the skin from being damaged by the absorbent. Further, in a mode in which the absorbent article 10 includes a function agent 18 (explained later), it is desirable to position the absorbent 50 in such a manner that the function agent 18 and the absorbent 50 are not in direct contact with each other.

In the packaged state, the absorbent 50 may be positioned between the stripping sheet 30 and the packaging sheet 20. Before the absorbent article 10 is used, the stripping sheet 30 is positioned between the absorbent 50 and the absorbent article 10. Accordingly, the absorbent 50 does not easily exert the capability thereof on the absorbent article 10 before use. It is therefore possible to prevent the adsorbing capability from being degraded. Further, because the absorbent 50 is interposed between the stripping sheet 30 and the packaging sheet 20, it is possible to prevent the absorbent 50 itself from being damaged at the time of unsealing the package 1. In the mode in which, like in the present embodiment, the fastening portions 15 are positioned only in the region overlapping with the absorbent core 13, the stripping sheet 30, the absorbent 50, and the packaging sheet 20 may be layered in the stated order from the skin side T1 toward the non-skin side T2. Further, in a modification example, in a mode in which flaps (which may be wings or hip flaps) are provided so as to spread outwardly in the width direction W relative to the absorbent core 13 while the flaps include the fastening portions, the flaps are folded onto the skin side T1 of the absorbent article 10 before use, and a flap stripping sheet is provided so as to cover the fastening portions on the flaps in the folded state. The absorbent 50 may be joined with the surface on the non-skin side of the flap stripping sheet (the surface on the side opposite from the absorbent article).

The absorbent 50 positioned between the stripping sheet 30 and the packaging sheet 20 may be joined with the packaging sheet 20, while not being joined with the stripping sheet 30. In the present embodiment, the absorbent 50 is joined with the inner surface (the surface on the stripping sheet side) of the packaging sheet 20 via a fixation portion 29. In the present aspect, at the time of taking the absorbent article 10 from the package 1, it is feared that the stripping sheet 30 might be pulled by one or more of the fastening portions 15 of the absorbent article 10 and that the stripping sheet 30 might jump out of the package space WS. In that situation, as a result of the absorbent 50 being joined with the packaging sheet 20, the absorbent 50 remains inside the package space WS. It is therefore possible to prevent the absorbent 50 from inadvertently falling. In addition, it is possible to have the adsorbing capability exerted on the absorbent article 10 after use that has been put in the package space WS.

In a modification example, the absorbent 50 positioned between the stripping sheet 30 and the packaging sheet 20 may be joined with the stripping sheet 30, while not being joined with the packaging sheet 20. Because the absorbent 50 is joined with the stripping sheet 30, it is possible to prevent the absorbent 50 from inadvertently falling. Further, by wrapping the absorbent article 10 after use with the stripping sheet 30, it is possible to have the adsorbing capability exerted better on the absorbent article 10 after use. In addition, because the absorbent 50 is not joined with the packaging sheet 20, the packaging sheet 20 easily gets loosened even when the package 1 is rubbed against while the package 1 is carried around or the like before the package 1 is unsealed. Thus, it is possible to prevent the package 1 from being damaged.

Furthermore, in another modification example, the absorbent 50 may be positioned in the package space WS without being joined. Because the absorbent 50 is not joined, the user is able to change the positioning of the absorbent 50 as appropriate in accordance with his/her manner of use and to use the absorbent 50 in a desired manner. More specifically, at the time of disposal, when the user uses a separate disposal-purpose member such as a disposal bag, toilet paper, or the like instead of using the packaging sheet, the user is able to use the absorbent 50 together with the disposal-purpose member. Alternatively, the user is also able to have the absorbent 50 placed on the absorbent article itself which has absorbed body fluid. Further, in the mode in which the absorbent 50 is not joined within the package space WS, the absorbent 50 may be positioned on the skin side T1 of the absorbent article 10. In the present aspect, the user is able to easily recognize the presence of the absorbent 50 before using the absorbent article 10 or the like. Consequently, the user is able to recognize the presence of the absorbent which will exert the adsorbing capability and to expect an effect on adverse impacts after the use, such as odor.

The absorbent 50 may be positioned so as to overlap with the non-joining region.

In other words, the absorbent 50 may be positioned in a region that does not overlap with the joining regions 25. In this situation, the absorbent 50 may be positioned so as to overlap with the non-joining region at least in the packaged state. At the time of taking the absorbent article 10 out of the package space WS, generally speaking, while a part of the absorbent article 10 is gripped, the absorbent article 10 is pulled to the outside of the package space WS. In that situation, together with the absorbent article 10, the stripping sheet 30 is pulled via the fastening portions 15. In the joining regions 25, the stripping sheet 30 is joined with the packaging sheet 20 and is thus unable to become apart from the packaging sheet 20. In the non-joining region, the stripping sheet 30 is able to become apart from the packaging sheet and thus moves toward the outside of the package space WS via the fastening portions 15. In the non-joining region, the stripping sheet 30 may easily become loose and rise. In the state after the absorbent article 10 is temporarily taken out, a space may easily be formed between the stripping sheet 30 and the packaging sheet 20. Because the absorbent 50 overlaps with the non-joining region, the adsorbing capability of the absorbent 50 is easily exerted. It is therefore possible to have the adsorbing capability exerted on the absorbent article 10 after use.

Between the first joining region 251 and the second joining region 252, the non-joining region may be provided in an uninterrupted manner. The fastening portions 15 and the absorbent 50 may be provided so as to overlap with the uninterruptedly-provided non-joining region. As shown in FIG. 4, when the user pulls while gripping either the front end or the rear end of the absorbent article 10, the stripping sheet 30 is pulled together with the absorbent article 10. In that situation, in the joining regions 25, the stripping sheet 30 is joined with the packaging sheet 20. In the regions overlapping with the joining regions 25 or in the vicinity thereof, the stripping sheet 30 is stripped from the fastening portions 15. However, on the package space WS side (the fold line FL side in the present embodiment) relative to the joining regions 25, the absorbent article 10 and the stripping sheet 30 are pulled, while the stripping sheet 30 and the fastening portions 15 remain joined with each other. In particular, in the mode in which the fastening portions 15 are provided on either side, in the front-rear direction L, of the fold line FL, the absorbent article 10 and the stripping sheet 30 are pulled on either side, in the front-rear direction L, of the fold line FL. In that situation, the non-joining region is provided between the first joining region 251 and the second joining region 252 in the uninterrupted manner, while the fastening portions 15 are provided so as to overlap with the non-joining region. As a result, the stripping sheet 30 is pulled together with the absorbent article 10 throughout the entire non-joining region. A space may easily be formed between the stripping sheet 30 and the packaging sheet 20, as a result of the stripping sheet 30 becoming loose and rise or the stripping sheet 30 jumping out of the package space WS. Further, because the non-joining region overlaps with the fold line FL, it is possible to position a fold line portion of the stripping sheet 30 to be apart from the packaging sheet 20. Because the absorbent 50 is provided so as to overlap with the non-joining region, it is possible to provide a wide space around the absorbent 50. Thus, when the absorbent article 10 after use is contained back into the package space WS, it is possible to have the adsorbing capability of the absorbent 50 exerted effectively.

The packaging sheet 20 may be a sheet member of which an oxygen transmission rate in the thickness direction T is equal to or lower than 200 cc/m2/day/atm. Because the packaging sheet 20 has the low oxygen transmission rate, it is possible to keep sealability of the package space WS in the packaged state and to prevent the adsorbing capability of the absorbent 50 from being degraded. It is therefore possible to have the adsorbing capability exerted on the absorbent article after use. As the packaging sheet described above, it is possible to use a sheet member formed by using, for example, EVOH resin (ethylene vinyl alcohol copolymer resin), PET (polyester), aluminum, polyvinyl alcohol, nylon, or the like. Examples of the packaging sheet 20 of the present embodiment include an aluminum laminated material (PET12/AL6/CPP30). It is possible to measure the oxygen transmission rate of the packaging sheet by implementing a well-known method such as a coulometric method (MOCON method) under the condition of 23°C and 0% RH.

The absorbent 50 may be positioned in a region that does not overlap with the fold line FL. In a mode in which the absorbent article 10 is folded while using a plurality of fold lines as reference points, the absorbent 50 may be positioned so as not to overlap with any of the fold lines. With this configuration, it is possible to prevent the absorbent 50 from being damaged from the folding that uses the fold line FL as a reference point. It is therefore possible to have the adsorbing capability exerted. Further, to maintain the state in which the fold line FL and the absorbent 50 do not overlap with each other, it is desirable to have the absorbent 50 joined with one of the packaging sheet 20 and the stripping sheet 30.

The absorbent article 10 may have the function agent 18. The function agent 18 includes at least one of: a testing member for testing a health condition of the wearer, an anti-bacterial agent, and an anti-virus agent. Before the absorbent article is used, the adsorbing capability of the absorbent 50 is able to prevent a capability of the function agent 18 from being degraded. The absorbent article 10 of the present embodiment includes the testing member serving as the function agent 18. In the present embodiment, because the moisture absorbent serving as the absorbent 50 adsorbs moisture, it is possible to prevent the testing member from reacting before use. The testing member may be, for example, a member that displays (a color indicating) the health condition of the wearer by using an indication drug. For example, it is possible to use the testing member disclosed in Japanese Patent Laid-Open No. 2020-022585. The testing member may be positioned between the topsheet 11 and the absorbent core 13. The anti-bacterial agent and the anti-virus agent may each be an inorganic compound (an inorganic anti-bacterial agent and an inorganic anti-virus agent). Generally speaking, because inorganic compounds are rarely harmful to living organisms, it is possible to enhance safety of the absorbent article, while enhancing anti-bacterial characteristics and anti-virus characteristics. Further, inorganic anti-bacterial agents and inorganic anti-virus agents are able to exert anti-bacterial functions and anti-virus functions thereof on more subjects than organic anti-bacterial agents and organic anti-virus agents are and are able to exert an effect on bacteria, mold, yeast, viruses, or the like. Further, inorganic anti-bacterial agents and inorganic anti-virus agents are able to sustain the anti-bacterial functions and the anti-virus functions thereof for longer periods of time than organic anti-bacterial agents and organic anti-virus agents are. Furthermore, inorganic anti-bacterial agents and inorganic anti-virus agents have lower possibilities of evaporating or being decomposed by water, heat, or the like and also have lower possibilities of being eluted by an organic solvent or the like. For these reasons, inorganic anti-bacterial agents and inorganic anti-virus agents can more easily be handled than organic anti-bacterial agents and organic anti-virus agents and are safer for living organisms. Thus, it is possible to enhance safety of the absorbent article 10 being contained. As the anti-bacterial/anti-virus agent, it is desirable to use, for example, a compound containing an inorganic substance such as Ag, Mn, Fe, Co, Ni, Cu, Zn, zeolite, titanium oxide, or the like. In a preferable example, an anti-bacterial agent and an anti-virus agent containing silver ions may be used.

Between the function agent 18 and the absorbent 50, the absorbent core 13 may be positioned. In the present embodiment, in the packaged state shown in FIG. 1, the testing member serving as the function agent 18 and the absorbent 50 overlap with each other in the thickness direction T, while the absorbent core 13 is positioned between the testing member and the absorbent 50. The absorbent core 13 includes an absorptive material such as pulp and easily functions as a buffer member. Because the absorbent core 13 positioned between the function agent 18 and the absorbent 50 functions as the buffer member, it is possible to prevent the function agent and the absorbent from inadvertently being damaged.

Next, an example of a method for manufacturing the package structured as described above will be explained. The method for manufacturing the package includes, at least, an adhesion application step, an absorbent joining step, and a sheet joining step. It is sufficient as long as the method for manufacturing the package includes, at least, the adhesion application step, the absorbent joining step, and the sheet joining step. For other manufacturing steps, it is possible to use a publicly known technique. In the adhesion application step, the plurality of joining regions having an adhesive agent applied thereto are provided on one of the stripping sheet 30 and the packaging sheet 20. The plurality of joining regions is positioned at intervals in a planar view. The absorbent joining step is a step performed later than the adhesion application step. Among the plurality of joining regions, the absorbent 50 is joined with a part of the joining regions. The part of the joining regions joined with the absorbent 50 may be one of the joining regions or may be two or more of the joining regions. The sheet joining step is a step performed later than the adhesion application step and the absorbent joining step. The stripping sheet 30 and the packaging sheet 20 are joined together via one or more other joining regions among the plurality of joining regions. In the present aspect, in the adhesion application step, it is possible to provide both the joining region with which the absorbent is joined and the joining region by which the stripping sheet and the packaging sheet are joined together. It is therefore possible to simplify the manufacturing steps as compared to another mode in which each of the joining regions is provided in a different one of separate steps. In the present embodiment, to one of the stripping sheet and the packaging sheet 20, an adhesive agent is applied in correspondence with the first joining region 251, the second joining region 252, and the fixation portion 29. Subsequently, the absorbent 50 is joined with the adhesive agent corresponding to the fixation portion 29. After that, via the adhesive agent corresponding to the first joining region 251 and the second joining region 252, the packaging sheet 20 and the stripping sheet 30 are joined together.

### (3) Second Embodiment (An embodiment of a container)

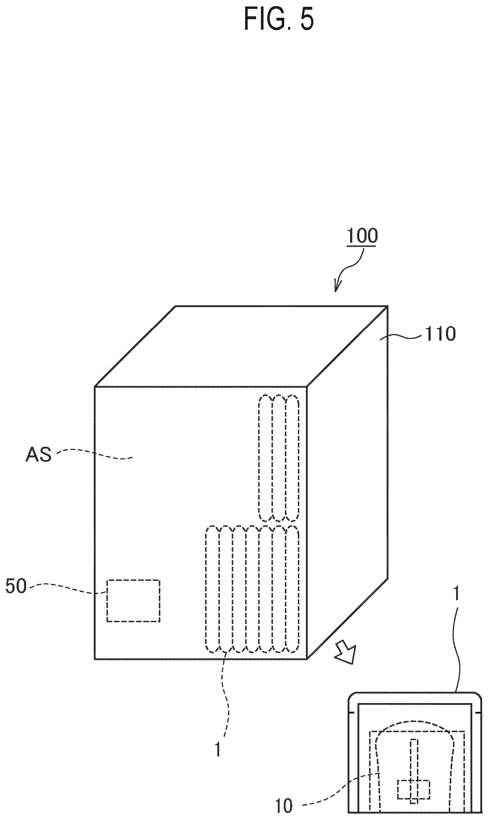

Next, a second embodiment will be explained. The second embodiment is related to a container 100. In the description of the embodiments below, some of the constituent elements that are the same as those in the first embodiment described above will be referred to by using the same reference characters, and explanations thereof will be omitted. FIG. 5 is a perspective view of the container 100. The container 100 includes absorbent articles 10, a container sheet 110 containing the plurality of absorbent articles 10 therein, and the absorbent 50. It is sufficient as long as each of the absorbent articles 10 includes the topsheet 11 that is liquid permeable, the backsheet 12 that is liquid impermeable, and the absorbent core 13 positioned between the topsheet 11 and the backsheet 12. The absorbent articles 10 of the second embodiment may each be the same as the absorbent article 10 of the first embodiment described above. The container sheet 110 contains the plurality of absorbent articles 10 in a containing space. The container sheet 110 may be formed to have a rectangular shape or a bag-like shape. To ensure sealability of the container 100, it is desirable that the container sheet 110 has low breathability. Examples of the container sheet 110 include film made of resin.

The container 100 is configured to be able to exert an adsorbing capability on the absorbent articles 10 after use. The container 100 includes the absorbent 50 of which adsorption is realized by at least one of an inorganic substance and an organic substance. The absorbent 50 is a separate body from the absorbent articles 10 and is provided in a containing space AS that, in the container 100, contains the absorbent articles 10 therein. It is general practice for some users to temporarily put a used absorbent article in the containing space AS, at the time of disposing of the absorbent article 10 after use. In that situation, because the absorbent 50 is provided in the containing space AS, it is possible to have the adsorbing capability exerted on the absorbent article 10 after use. Further, the absorbent 50 is a separate body from the absorbent articles 10 and, during use, exerts no adsorbing capability on the body fluid absorbed by the absorbent articles 10. Consequently, it is possible to have the adsorbing capability exerted on the absorbent articles 10 after use that are positioned in the containing space AS.

The container sheet 110 may be a sheet member of which the oxygen transmission rate in the thickness direction T is equal to or lower than 200 cc/m²/day/atm. In the present aspect, because the container sheet 110 has the low oxygen transmission rate, it is possible to keep sealability of the containing space AS, to prevent the adsorbing capability of the absorbent 50 from being degraded, and to have the adsorbing capability exerted better on the absorbent articles 10 after use.

It is sufficient as long as the absorbent 50 is configured so that adsorption is realized by at least one of an inorganic substance and an organic substance. The absorbent 50 may be the same as that of the first embodiment. Further, the container 100 may contain, in the containing space AS, the package 1 individually packaged by the packaging sheet 20. Alternatively, the container 100 may contain, in the containing space AS, a layered body in which the absorbent articles 10 are layered with one another without being individually packaged by the packaging sheet 20. In a preferable example, when what is contained in the containing space AS is in the form of the packages 1, because the absorbent 50 does not easily function on the absorbent articles 10 before the absorbent articles 10 are used, it is possible to have the adsorbing capability of the absorbent 50 exerted better after the absorbent articles 10 are used.

Each of the absorbent articles 10 of the second embodiment may be an absorbent article (an absorbent article including the absorbent 50) according to a third embodiment or a fourth embodiment described below or may be an absorbent article (an absorbent article not including the absorbent 50) different from that of the third embodiment or the fourth embodiment. Further, in a mode in which the package 1 is contained in the containing space AS, the package may be the package (the package including the absorbent that is a separate body from the absorbent article) according to the first embodiment or may be a package (a package that does not include an absorbent that is a separate body from the absorbent article) different from that of the first embodiment.

### (4) Third Embodiment and Fourth Embodiment (Embodiments of absorbent articles)

Next, the third embodiment and the fourth embodiment will be explained. The third embodiment and the fourth embodiment are related to absorbent articles 10X and 10Y each including the absorbent 50. It is sufficient as long as the absorbent 50 is configured so that adsorption is realized by at least one of an inorganic substance and an organic substance. The absorbent 50 may have the same configuration as that in the first embodiment or the like. Further, because the absorbent of the third embodiment and the fourth embodiment is not a separate body from the absorbent article 10 but is provided in the absorbent article 10, it is desirable that the absorbent has a shape that does not easily cause discomfort during use of the absorbent article. For example, the absorbent may have a sheet-like shape or may have a spherical shape. The absorbent does not necessarily need to be solid and may be applied as liquid or fluid. The absorbent 50 may be configured by a sheet (a topsheet, a backsheet, a core wrap sheet, or the like) structuring the absorbent article.

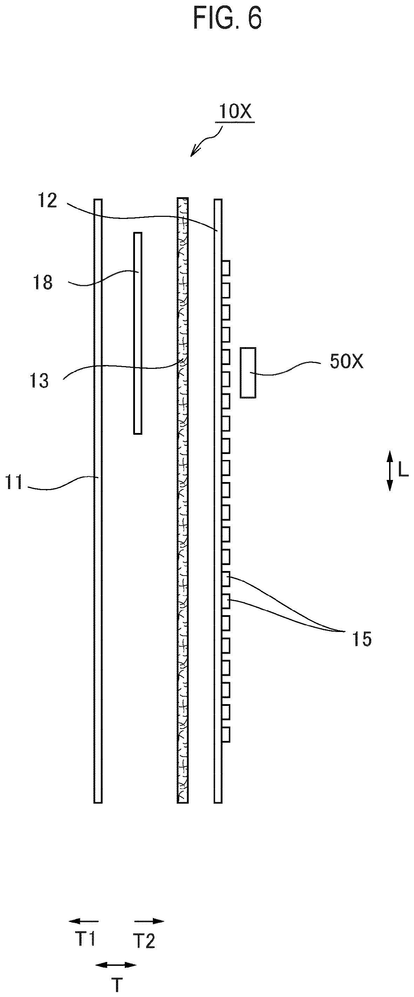

With reference to FIG. 6, the absorbent article 10X according to the third embodiment will be explained. FIG. 6 is a cross-sectional view of the absorbent article 10X, using a cross-sectional plane taken along line B-B indicated in FIG. 2 as reference. The absorbent article 10X includes an absorbent 50X located on the non-skin side T2 relative to the backsheet 12. The absorbent of the present embodiment has a sheet-like shape. The absorbent 50X is joined with a part of the adhesive agent structuring the fastening portions 15 and is joined with the absorbent article via the adhesive agent.

The absorbent 50X is positioned on the non-skin side relative to the liquid-impermeable backsheet 12. Excreted body fluid is guided from the topsheet 11 to the absorbent core 13 and is absorbed by the absorbent core 13. Positioned on the non-skin side T2 relative to the absorbent core 13 is the liquid-impermeable backsheet 12. In other words, during the use of the absorbent article 10, the body fluid is not easily guided to the non-skin side T2 of the backsheet 12. For being positioned in such a manner that the absorbent core 13 and the backsheet 12 are interposed, the absorbent 50 does not easily come into contact with the body fluid during use and does not easily exert the adsorbing capability. Consequently, also in the absorbent article 10X after use, the adsorbing capability of the absorbent 50X is prevented from being degraded. It is therefore possible to have the adsorbing capability exerted.

The function agent 18 may be positioned on the skin side T1 relative to the backsheet 12. The function agent 18 and the absorbent 50X are positioned while the backsheet 12 is interposed therebetween. With the backsheet 12, it is possible to avoid the situation where the function agent 18 comes into contact with the absorbent 50X. In addition, because the backsheet 12 is liquid impermeable, it is also possible to avoid passing of moisture such as body fluid. Accordingly, it is possible to maintain the capability of the function agent 18 and the capability of the absorbent 50X. Consequently, the function agent 18 is able to exert the capability thereof before the use, whereas the absorbent 50X is able to exert the capability thereof after the use.

The absorbent 50X may be a member structuring the non-skin surface of the absorbent article 10X. In other words, the absorbent article 10X does not need to include a member that covers the non-skin side T2 of the absorbent 50X. With this configuration, the adsorbing capability of the absorbent 50X is easily exerted, while the non-skin surface of the absorbent article 10 is facing out. For example, at the time of disposal of the absorbent article 10, the adsorbing capability is easily exerted while the absorbent article 10 is in a rolled-up state with the skin side T1 in. In addition, when the absorbent article 10X has the fastening portions 15 like in the present embodiment, because the absorbent 50X is interposed between the backsheet 12 and a wearable article during the use of the absorbent article, it is possible to prevent deterioration or damage thereof.

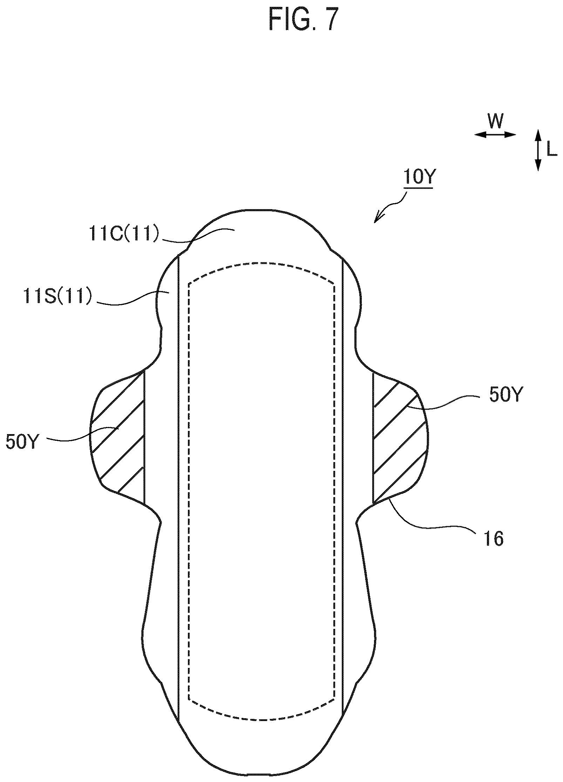

Next, with reference to FIG. 7, the absorbent article 10Y of the fourth embodiment will be explained. FIG. 7 is a plan view of the absorbent article 10Y according to the fourth embodiment as seen from the skin side. The absorbent article 10Y has wings 16 realized as flaps. The absorbent 50Y is accompanying the wings 16. It is sufficient as long as the wings 16 are configured so as to be folded back onto the non-skin side of the absorbent article, at the time of wearing. The wings 16 may be configured so as to be fastened to the non-skin surface of a wearable article. In the fourth embodiment, the absorbent 50Y is positioned with the wings 16. The wings 16 are folded back onto the non-skin side T2 of the absorbent article 10 during use. The absorbent 50Y does not easily come into contact with body fluid during use and does not easily exert the adsorbing capability. Consequently, also in the absorbent article 10 after use, the adsorbing capability of the absorbent 50Y is prevented from being degraded. It is therefore possible to have the adsorbing capability exerted. Further, although the wings are provided as the flaps in the fourth embodiment, the flaps do not necessarily need to be wings. It is sufficient as long as the flaps are positioned on the non-skin side T2 relative to the backsheet 12 at the time of use. The flaps may be flap portions which are provided for a tape-type disposable diaper and to which a fastening tape is fastened. The flaps may be positioned on the non-skin side T2 of the backsheet as a result of being folded back onto the non-skin surface side of the absorbent article at the time of wearing or may be positioned on the non-skin side T2 of the backsheet as a result of being positioned so as to cover the non-skin surface of the absorbent article without being folded back onto the non-skin surface side of the absorbent article at the time of wearing.

As the topsheet, the absorbent article of the present embodiment includes: a center sheet 11C positioned at the center, in the width direction, of the absorbent core; and side sheets 11S covering outer portions of the center sheet and spreading outwardly in the width direction W relative to the center sheet 11C. The absorbent is applied to the sheets (the side sheets) structuring the skin surface of the wings. Further, as another example of the absorbent, the absorbent 50 may be a separate body from the absorbent article, like in the first embodiment, so that the absorbent being the separate body is fixed to the flaps.

Only the wings 16 may be provided with the absorbent 50Y. In other words, the absorbent 50Y may be provided only in the regions that are folded back onto the non-skin side of the absorbent article at the time of use. In the present aspect, because only wings 16, which does not easily come into contact with body fluid during use, are provided with the absorbent 50Y, it is possible to prevent the capability from being exerted during use of the absorbent article 10. Consequently, it is possible to have the adsorbing capability of the absorbent exerted after the use.

Before use, the wings 16 may be folded so as to cover the skin-side surface of the absorbent article. At least a part of the absorbent 50Y may be positioned on the skin surfaces of the wings 16 in the folded regions of the wings 16. Before the absorbent article is used, the absorbent 50Y is interposed between each of the folded regions of the wings 16 and an unfolded region of the absorbent article. It is therefore possible to prevent the absorbent 50Y from being deteriorated or falling before use. Further, in a mode in which the function agent 18 is provided, at least a part of the function agent 18 may be covered by the folded regions of the wings 16. With this configuration, before the absorbent article is used, it is possible to protect the function agent 18 with the wings.

In the absorbent article 10Y, the absorbent 50Y may be positioned on the skin side relative to the topsheet 11. Alternatively, the absorbent 50Y may be positioned with the topsheet 11. The absorbent 50Y may be a member structuring the skin surface of the absorbent article. In other words, the absorbent article 10Y does not necessarily need to include a member that covers the skin side T1 of the absorbent 50Y. In the present aspect, the user is able to easily recognize the presence of the absorbent 50Y before use and during wearing. Consequently, the user is able to recognize the presence of the absorbent 50Y which will exert the adsorbing capability and to expect an effect on adverse impacts after the use, such as odor.

### (5) Other Embodiments

Although the present invention has been described in detail above using the aforementioned embodiments, it is clear to those skilled in the art that the present invention is not limited to the embodiments described in this specification. The present invention can be implemented as modified and changed aspects without departing from the spirit and scope of the present invention as defined by the description of the claims. Therefore, the description in this specification is for the purpose of illustrative explanation and does not have any restrictive meaning for the present invention.

It is also acceptable to apply the same configuration as that of the absorbent article 10 of the first embodiment, to the absorbent articles in the container of the second embodiment, the absorbent article 10X of the third embodiment, and the absorbent article 10Y of the fourth embodiment. For example, any of the absorbent articles may include the function agent. During use of the absorbent article and in the packaged state before use, the absorbent core may be positioned between the function agent and the absorbent.

Further, in the first to the fourth embodiments, the absorbent 50 may be provided in a position that does not overlap with the function agent 18. When the function agent 18 and the absorbent 50 each have a thickness, if the function agent 18 overlapped with the absorbent 50, there might be a portion formed to be thicker than the surroundings thereof. In that situation, external force might easily be applied to the thicker portion at the time of manufacturing or during transport. However, because the absorbent 50 is provided in the position that does not overlap with the function agent 18, it is possible to prevent external force from being applied locally and to have the functions of the function agent 18 and the absorbent 50 exerted.

In the first to the fourth embodiments, the absorbent 50 may be provided in a position that does not overlap with a post-process tape for maintaining the absorbent article in a rolled state at the time of disposal or a hook member for maintaining the shape of the absorbent article at the time of disposal or wearing. Because the absorbent is provided in the position that does not overlap with the post-process tape or the hook member, it is possible to prevent external force from being applied locally and to have the functions of the function agent 18 and the absorbent 50 exerted.

It is also acceptable to apply the same configuration as that of the absorbent 50 of the first embodiment, to the absorbent in the container of the second embodiment, the absorbent 50X of the third embodiment, and the absorbent 50Y of the fourth embodiment. Further, the absorbent may be positioned in a region that does not overlap with a fold line used for folding the absorbent article. The quantity of the fold lines may be one like in the first embodiment or may be multiple (two or more). In the mode in which the plurality of fold lines is formed, the absorbent does not need to be folded at any of the fold lines.

Further, the dimension of the absorbent 50 (in the mode in which an adsorption bag is provided, the dimension including the adsorption bag) may be smaller than the dimension of the stripping sheet 30 in the width direction W. In the aspect in which the dimension of the absorbent 50 is smaller than the dimension of the stripping sheet 30 in the width direction W, when the absorbent 50 is positioned on the skin side T1 relative to the stripping sheet 30, because the absorbent 50 is not easily visually recognized from the outside of the package, the user does not easily notice the presence of the absorbent 50. The dimension of the absorbent 50 may be in the range of 10 mm to 30 mm by 20 mm to 50 mm. It is desirable to configure the absorbent 50 so that the spherical bodies 51 themselves that exert the adsorption effect are prevented from falling out of the adsorption bag 52. For this reason, it is desirable that the adsorption bag 52 does not have a structure that may lead to the falling, such as a hole, an incision, a vulnerable part, or the like.

The entire content of Japanese Patent Application No. 2023-040748, filed on March 15, 2023, is incorporated herein by reference.

### [INDUSTRIAL APPLICABILITY]

According to the present invention, it is possible to provide an absorbent article that can exert an adsorbing capability after the absorbent article is used.

### [REFERENCE SIGNS LIST]

1: Package 10: Absorbent article 10X: Absorbent article 10Y: Absorbent article 11: Topsheet 12: Backsheet 13: Absorbent core 15: Fastening portion 16: Wing (Flap) 18: Functional agent 20: Packaging sheet 25: Joining region 251: First joining region 252: Second joining region 30: Stripping sheet 50, 50X, 50Y: Adsorbent 100: Housing member 110: Containing sheet AS: Containing space FL: Fold line L: Front-rear direction T: Thickness direction T1: Skin side T2: Non-skin side W: Width direction WS: Packaging space

## Claims

1. A package comprising:
an absorbent article including a liquid-permeable topsheet, a liquid-impermeable backsheet, and an absorbent core positioned between the topsheet and the backsheet; and
a packaging sheet that individually packages the absorbent article, wherein
the package includes an absorbent of which adsorption is realized by at least one of an inorganic substance and an organic substance, and
the absorbent is a separate body from the absorbent article and is provided in a package space that, in the package, contains the absorbent article therein.

2. The package according to claim 1, wherein
the absorbent article has a fastening portion to be fastened to a wearable article when being worn,
the package includes a stripping sheet that is positioned between the absorbent article and the packaging sheet and that covers the fastening portion before the absorbent article is used, and
the absorbent is positioned between the stripping sheet and the packaging sheet.

3. The package according to claim 2, wherein
the absorbent is joined with the packaging sheet, while not being joined with the stripping sheet.

4. The package according to claim 2, wherein
the absorbent is joined with the stripping sheet, while not being joined with the packaging sheet.

5. The package according to any one of claims 2 to 4, wherein
a region in which the packaging sheet overlaps with the stripping sheet is provided with a joining region in which the packaging sheet and the stripping sheet are joined together and a non-joining region in which the packaging sheet and the stripping sheet are not joined together, and
the absorbent is positioned so as to overlap with the non-joining region.

6. The package according to claim 5, wherein
the absorbent article is folded in two while using a fold line extending in a width direction as a reference point,
an outlet through which the absorbent article can be taken out is configured in the package so as to be provided at an end positioned opposite from the fold line, which is one of ends of the package in a front-rear direction,
the joining region includes a first joining region and a second joining region positioned on either side, in the front-rear direction, of the fold line,
the non-joining region is provided between the first joining region and the second joining region in an uninterrupted manner, and
the fastening portion and the absorbent are provided so as to overlap with the non-joining region.

7. The package according to claim 1 or 2, wherein
the packaging sheet is a sheet member of which an oxygen transmission rate in a thickness direction is equal to or lower than 200 cc/m²/day/atm.

8. The package according to claim 1 or 2, wherein
before use, the absorbent article is folded while using a fold line as a reference point, and the absorbent is positioned in a region that does not overlap with the fold line.

9. The package according to claim 1 or 2, wherein
the absorbent article includes a function agent including at least one of: a testing member for testing a health condition of a wearer, an anti-bacterial agent, and an anti-virus agent.

10. The package according to claim 9, wherein
the absorbent core is positioned between the function agent and the absorbent.

11. A container comprising:
a plurality of absorbent articles each including a liquid-permeable topsheet, a liquid-impermeable backsheet, and an absorbent core positioned between the topsheet and the backsheet; and
a container sheet containing therein the plurality of absorbent articles, wherein
the container includes an absorbent of which adsorption is realized by at least one of an inorganic substance and an organic substance, and
the absorbent is a separate body from the absorbent article and is provided in a containing space that, in the container, contains the absorbent articles therein.

12. The container according to claim 11, wherein
the container sheet is a sheet member of which an oxygen transmission rate in a thickness direction is equal to or lower than 200 cc/m²/day/atm.

13. An absorbent article comprising:
a liquid-permeable topsheet, a liquid-impermeable backsheet, and an absorbent core positioned between the topsheet and the backsheet, wherein
the absorbent article includes an absorbent of which adsorption is realized by at least one of an inorganic substance and an organic substance, and
the absorbent is provided on a non-skin side relative to the backsheet.

14. The absorbent article according to claim 13, wherein
the absorbent article includes a function agent including at least one of: a testing member for testing a health condition of a wearer, an anti-bacterial agent, and an anti-virus agent, and
the function agent is positioned on a skin side relative to the backsheet.

15. The absorbent article according to claim 13 or 14, wherein
the absorbent is a member structuring a non-skin surface of the absorbent article.

16. An absorbent article comprising:
a liquid-permeable topsheet, a liquid-impermeable backsheet, and an absorbent core positioned between the topsheet and the backsheet, wherein
the absorbent article includes an absorbent of which adsorption is realized by at least one of an inorganic substance and an organic substance,
the absorbent article has a flap positioned on a non-skin side relative to the backsheet when being worn, and
the flap is provided with the absorbent.

17. The absorbent article according to claim 16, wherein
only the flap is provided with the absorbent.

18. The absorbent article according to claim 17, wherein
the flap is folded so as to cover a skin-side surface of the absorbent article before use, and
at least a part of the absorbent is positioned on a skin surface of the flap in a folded region of the flap.

19. A method for manufacturing a package including an absorbent article having a fastening portion to be fastened to a wearable article when being worn, a stripping sheet that covers the fastening portion, and a packaging sheet that individually packages the absorbent article while being joined with a surface of the stripping sheet positioned opposite from the absorbent article, the method comprising:
an adhesion application step of providing one of the stripping sheet and the packaging sheet with a plurality of joining regions each having an adhesive agent applied thereto;
an absorbent joining step of joining the absorbent with a part of the plurality of joining regions; and
a sheet joining step of joining the stripping sheet and the packaging sheet together, via one or more other joining regions among the plurality of joining regions.
